(19) Europäisches Patentamt
European Patent Office
Office européen des brevets

(11) EP 0 583 566 B1

(12) EUROPEAN PATENT SPECIFICATION

(45) Date of publication and mention of the grant of the patent:
**23.09.1998 Bulletin 1998/39**

(51) Int Cl.[6]: **C07J 41/00**, A61K 31/575 // C07J9/00

(21) Application number: **93109377.7**

(22) Date of filing: **11.06.1993**

# (54) Process for the preparation of glycine-conjugated bile acids

Verfahren zur Herstellung Glycin-konjugierter Gallensäure

Procédé de préparation des acides biliaires conjugés à glycine

(84) Designated Contracting States:
**AT BE CH DE DK ES FR GB GR IE IT LI LU MC NL PT SE**

(30) Priority: **04.08.1992 IT MI921924**

(43) Date of publication of application:
**23.02.1994 Bulletin 1994/08**

(73) Proprietor: **ERREGIERRE INDUSTRIA CHIMICA Spa**
**I-24060 San Paolo d'Argon (BG) (IT)**

(72) Inventors:
- **Bonaldi, Antonio**
  **I-24060 Chiuduno (IT)**
- **Molinari, Egidio**
  **I-22030 Longone Al Segrino (Como) (IT)**
- **Roda, Aldo**
  **I-40136 Bologna (IT)**

(74) Representative: **Gervasi, Gemma, Dr. et al**
**NOTARBARTOLO & GERVASI Srl,**
**Corso di Porta Vittoria, 9**
**20122 Milano (IT)**

(56) References cited:
**EP-A- 0 100 983**

- **J. LIPID RES., vol. 22, no. 2, 1981 pages 254-270, H. IGIMI ET AL. 'Cholesterol gallstone dissolution in bile: dissolution kinetics of crystalline (anhydrate and monohydrate) cholesterol with chenodeoxycholate, ursodeoxycholate and their glycine and taurine conjugates.'**
- **Z. GASTROENTEROLOGIE, vol. 19, no. 4, 1981 pages 159-163, R. RAEDSCH ET AL. 'Kinetics of cholesterol gallstone dissolution by glycocheno-,glycoursodeoxycholic acid, end mixtures thereof in vitro.'**
- **DATABASE WPI Week 7616 Derwent Publications Ltd., London, GB; AN 76-29269x & JP-A-51 026 870 (EISAI KK) , 6 March 1976 & JP-A-58 026 360**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to a process for the preparation of glycine-conjugated bile acids and/or pharmaceutically acceptable salts thereof.

### PRIOR ART DICLOSURE

The amides formed by bile acids with the amino function of the amino acids, glycine and taurine, are known as conjugated bile acids and relative bile salts.

The bile acids secreted by the liver are almost entirely in the conjugated form. For example, glycoursodeoxycholic acid is the major metabolite that is present in high amounts in bile after ursodeoxycholic acid prolonged administration.

The synthesis of bile acids conjugated with taurine and glycine was described by Bergström and Norman (Enciclopedia della Chimica, USES, Firenze, pages 421-426).

According to said procedure, a mixed anhydride was obtained by treating a bile acid salt and tributylamine with ethylchlorocarbonate in dioxane at low temperature. The obtained anhydride was reacted with a glycine or taurine sodium salt aqueous solution to produce the sodium salt of the conjugated acid. However, the product yields and, especially, purity obtained by the aforesaid process are not high.

The Applicant has surprisingly found a process for the preparation of conjugated bile acids of general formula (I) that is not adversely affected by the disadvantages inherent in the known process.

Like the process known from literature, the process of the present invention comprises:

a) optionally salifying bile acid of formula (II)

(II) Y-OH

wherein Y is the acyl radical of a bile acid selected from the group consisting of ursodeoxycholic, chenodeoxycholic, lithocholic, 3$\alpha$,7$\beta$,12$\alpha$-trihydroxycholanic, 3$\alpha$,7$\beta$-dihydroxy-12-hetocholanic, deoxycholic, dehydrocholic hyodeoxycholic and hyocholic acids, with a tertiary amine of alkyl or heteroaromatic type in an aprotic solvent at a temperature below 20°C;

b) reacting either the mixture obtained in (a) containing the aforesaid bile acid salt or previously isolated salt, with a chloroformate of general formula (III):

(III) Cl-COOR

where R is selected from the group consisting of $C_1$-$C_5$ alkyl, phenyl, benzyl, at a temperature below 20°C in the presence of an aprotic solvent to give the corresponding mixed anhydride of formula (IV):

(IV) Y-O-COOR

where Y and R are as defined above.

But, unlike the process known from literature, the process as per the present invention is characterized by the following stages:

c) mixed anhydride (IV) is reacted with a phenol of general formula (V)

(V)

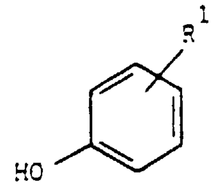

where $R^1$ is selected from H and $C_1$-$C_5$ alkyl, $C_2$-$C_5$ acyl and nitro group, at a temperature below 60°C in the presence of an aprotic solvent and of a tertiary amine as per (a) to give the corresponding phenol ester of formula (VI):

(VI)

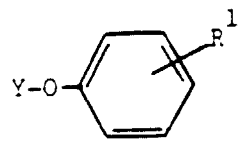

where Y and $R^1$ are as defined above;

d) the product is separated by water addition, extraction of phenol ester in a solvent, evaporation and crystallization, followed by an optional recrystallization;

e) phenol ester is treated with an aqueous solution of glycine as such or in the form of an alkaline metal salt or of a tertiary amine of alkyl or heteroaromatic type, at a temperature ranging from 0°C to 100°C (ammonolysis) optionally in the presence of a protic solvent;

f) the product is separated precipitating by acidifying the reaction mixture produced in (e) to a pH between 1 and 4, after optional solvent evaporation and relative filtration;

g) the product recovered in (f) is crystallized in a protic or aprotic polar solvent.

The process of the present invention - which envisages the essential stages of producing an intermediate, i.e. phenol ester (VI), and removing same from the reaction mixture by crystallization - permits to obtain the desired product in high yields and in high purity degree.

**DETAILED DESCRIPTION OF THE INVENTION**

The solvent used in stages (a) and (b) of the process of the present invention may be either an aprotic polar solvent, preferably acetone, ethyl acetate, dioxane, tetrahydrofuran, or an aprotic dipolar solvent, such as dimethylformamide.

The intermediates obtained in stages (a) and (b) may be either isolated or not: in the latter case the solvent used in stage (a) of the process under the invention is the same as that used in stage (b).

The tertiary amines used in stages (a) and (c) to give the bile acid salt and phenol salt, respectively, are preferably selected from triethylamine, tributylamine, and pyridine.

The $C_1$-$C_5$ alkyl chloroformate (III) used in (b) is preferably a methyl or an ethyl.

The aprotic solvent used in (c) may be an aprotic polar solvent selected from acetone, ethyl acetate, dioxane, tetrahydrofuran, or an aprotic dipolar solvent, i.e. N,N-dimethylformamide.

The solvent used to crystallize the phenol ester (VI), stage (d) of the process of the present invention, is a polar solvent, either protic or aprotic, such as a $C_1$ to $C_4$ alcohol, or an aprotic polar solvent, as for example acetone and acetic acid esters with $C_1$ to $C_4$ alcohols, preferably acetonitrile either alone or mixed with the aforesaid alcohols.

The bases used to obtain the glycine salt in stage (e) of the process of the present invention are selected out of: sodium hydroxide, potassium hydroxide, lithium hydroxide, potassium and sodium carbonate or bicarbonate, as well as the tertiary alkyl amines or pyridine preferably used in stage (a) for bile acid salt production.

The solvent used in the aforementioned ammonolysis - stage (e) of the process of the present invention - is a polar solvent, such as water either alone or mixed with $C_1$ to $C_4$ alcohol.

The acid used in stage (f) for conjugated raw acid (I) precipitation is a mineral acid, such as hydrochloric, sulphuric, and phosphoric acids, or an organic acid such as acetic acid.

The solvent used in stage (g) of the process of the present invention for the conjugated bile acid (I) crystallization is a protic polar solvent, such as an alcohol, and preferably ethanol, isopropanol, sec-butanol, or an aprotic polar solvent, such as acetone, or $C_1$-$C_4$ alcohol acetates and preferably ethyl acetate.

The following examples are reported by way of indication, not of limitation.

**EXAMPLE 1**

Ursodeoxycholic acid (250 g) was suspended in 1000 ml dioxane and treated with 66 g triethylamine. The mixture was stirred at room temperature for 1 hr. After cooling to 10°C, 70 g ethyl chloroformate was added dropwise at a temperature maintained below 20°C. Once the addition was completed, the mixture was stirred at 15°C-20°C for 1 hr, poured into a solution prepared separately by dissolving 150 ml p-hydroxypropiophenone in 500 ml ethyl acetate and 101 g triethylamine and by heating the obtained solution to 35°C-40°C, which temperature was maintained during the dropwise addition and for one or two more hours.

The addition of deionized water (1000 ml) gave two phases.

The organic phase was washed with 500 ml deionized water and evaporated to a thick oil, which was crystallized with 1500 ml acetonitrile by heating and subsequent cooling to 15°C.

The precipitate was separated by filtration and washed thoroughly with acetonitrile.

Drying yielded 300 g ursodeoxycholic acid ester with the following characteristics: crystalline white powder

m.p. = 88 - 92°C
$[\alpha]_D^{20}$ = + 41.5°

Ammonolysis: 65 g glycine and 34 g sodium hydroxide were dissolved in deionized water. The solution was added with 280 ml secondary butyl alcohol and 300 g phenol ester obtained as described above.

The solution was refluxed for 5 hrs, cooled to 30°C. Then the product was precipitated by acidification with phosphoric acid to a pH ranging from 2.5 to 3. After cooling to 15°C, filtering and washing with water and sec-butanol, the moist product was crystallized by hot dissolution with sec-butanol, followed by cooling, filtering and washing with sec-butanol. 260 g glycoursodeoxycholic acid with the following characteristics were obtained:
crystalline white powder

m.p. = 230 - 233°C
$[\alpha]_D^{20}$ = + 54.6° (C: 2% in methanol)
Titre = 99.82%

**EXAMPLE 2**

The phenol ester was obtained following the same procedure as per Example 1, but using ethyl acetate instead of dioxane as the reaction solvent for mixed anhydride preparation.

310 g phenol ester with the following characteristics was obtained:

m.p. = 89 - 92°C
$[\alpha]_D^{20}$ = + 42.1°

Ammonolysis was carried out according to the same procedure as per Example 1.

The product was recrystallized by boiling glycoursodeoxycholic acid with ethyl acetate and water, followed by cooling, filtering, washing with water and ethyl acetate, and drying.

265 g glycoursodeoxycholic acid with the following characteristics was obtained:
crystalline white powder

m.p. = 231.5 - 232.5°C
$[\alpha]_D^{20}$ = + 55° (C: 2% in methanol)
Titre = 99.9%

By using the same basic procedure and using the solvents, bases, and chloroformates indicated above, the following glycine-conjugated bile acids were obtained: glycochenodeoxycholic, glycolithocholic, glyco-3α-7β-12α-trihydroxycholanic, glyco-3α-7β-dihydroxy-12-ketocholanic, glycodeoxycholic, glycodehydrocholic, glycohyodeoxycholic,

glycohyocholic.

## Claims

1. A process for the preparation of glycine-conjugated bile acids of general formula (I):

(I) $Y\text{-}NH\text{-}CH_2\text{-}COOH$

wherein Y is the acyl radical of a bile acid selected from the group consisting of: ursodeoxycholic, chenodeoxycholic, lithocholic, 3α-7β-12α-tri-hydroxycholanic, 3α-7β-dihydroxy-12-ketocholanic, deoxycholic, dehydrocholic, hyodeoxycholic, hyocholic acids, comprising:

a) optionally salifying bile acid of formula (II)

(II) Y-OH

wherein Y is as defined above, with a tertiary amine of alkyl or heteroaromatic type in an aprotic solvent at a temperature below 20°C;

b) treating the reaction mixture containing the aforesaid bile acid salt, or previously isolated salt, with a chloroformate of general formula (III):

(III) Cl-COOR

where R is selected from the group consisting of $C_1$-$C_5$ alkyl, phenyl, benzyl, at a temperature below 20°C in the presence of an aprotic solvent to give the corresponding mixed anhydride of formula (IV)

(IV) Y-O-COOR

where Y and R are as defined above;

c) reacting the mixed anhydride (IV) with a phenol of general formula (V):

(V)

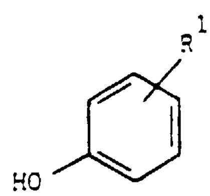

where $R^1$ is selected from H and $C_1$-$C_5$ alkyl, $C_2$-$C_5$ acyl and nitro group, at a temperature below 60°C in the presence of an aprotic solvent and of a tertiary amine to give the corresponding phenol ester of formula (VI):

(VI)

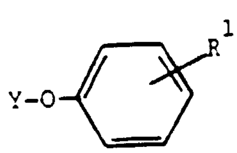

d) separating the product by water addition, phenol ester extraction in a solvent, evaporation and crystallization, followed by an optional recrystallization;

e) treating the phenol ester with an aqueous solution of glycine as is or in the form of an alkaline metal salt or

of a tertiary amine of an alkyl or heteroaromatic type, at a temperature ranging from 0°C to 100°C optionally in the presence of a protic solvent;
f) separating the product, precipitating by acidifying the reaction mixture produced in (e) to a pH between 1 and 4, after optional solvent evaporation and relative filtration;
g) the product recovered in (f) is crystallized in a protic or aprotic polar solvent.

**2.** The process according to claim 1 wherein the aprotic solvent used in stages (a) and (b) is an aprotic polar solvent selected from acetone, ethyl acetate, dioxane, tetrahydrofuran, or an aprotic dipolar solvent, selected from dimethylformamide.

**3.** The process according to claim 1 wherein the intermediates obtained in (a) and (b) are not isolated.

**4.** The process according to claim 1 wherein the tertiary amines used in (a) and (c) to give the bile acid salt and phenol salt, respectively, are selected from triethylamine, tributylamine, and pyridine.

**5.** The process according to claim 1 wherein $C_1$-$C_5$ alkyl chloroformate (III) used in (b) is methyl or ethyl chloroformate.

**6.** The process according to claim 1 wherein the aprotic solvent used in (c) is an aprotic polar solvent selected from acetone, ethyl acetate, dioxane, tetrahydrofuran, or an aprotic dipolar solvent, such as N,N-dimethylformamide.

**7.** The process according to claim 1 wherein the phenol ester crystallization solvent, stage d, is a protic or aprotic polar solvent, preferably acetonitrile either alone or mixed with $C_1$-$C_4$ alcohols.

**8.** The process according to claim 2 wherein the bases used to obtain the glycine salt employed in stage (e) are selected from: sodium hydroxide, potassium hydroxide, lithium hydroxide, potassium or sodium carbonate or bicarbonate, and the tertiary amines are selected from triethylamine, tributylamine, and pyridine.

**9.** The process according to claim 1 wherein the acid used in stage (f) for conjugated raw acid (I) precipitation is a mineral acid, selected from hydrochloric, sulphuric, and phosphoric acids, or an organic acid such as acetic acid.

**10.** The process according to claim 1 wherein the solvent used for conjugated bile acid (I) crystallization, stage g, is a protic polar solvent consisting of an alcohol or an aprotic polar solvent consisting of acetone or $C_1$-$C_4$ alcohol acetates.

**Patentansprüche**

**1.** Verfahren zur Herstellung Glyzin-konjugierter Gallensäure der allgemeinen Formel (I):

(I) $Y\text{-}NH\text{-}CH_2\text{-}COOH$

in der Y das Acyl-Radikal einer Gallensäure ist, die aus der aus Ursodesoxycholinsäure, Chenodesoxycholinsäure, Lithocholinsäure, 3α-7β-12α-tri-hydroxycholanesäure, 3α-7β-Dihydroxy-12-Ketocholanesäure, Desoxycholinsäure, Dehydrocholinsäure, Hyodesoxycholinsäure, Hyocholinsäure bestehenden Gruppe ausgewählt wird, bestehend aus:

a) wahlfreie Umsetzung in Salz der Gallensäure der Formel (II)

(II) Y-OH

in der Y wie oben ist, mit einem tertiären Amin von Alkyl- oder heteroaromatischem Typ in einem aprotischen Lösungsmittel bei einer Temperatur unter 20°C;
b) Behandlung des obengenannten Gallensäure-Salzes, oder der das vorher isolierte Salz umfassenden Reaktionsmischung mit einem Chloroformiat der allgemeinen Formel (III):

(III) Cl-COOR

in der R aus der aus $C_1$-$C_5$ Alkyl, Phenyl, Benzyl bestehenden Gruppe ausgewählt wird, bei einer Temperatur unter 20°C in Anwesenheit eines aprotischen Lösungsmittels, um das entsprechende gemischte Anhydrid der Formel (IV)

(IV) Y-O-COOR

zu erhalten, in der Y und R wie oben sind;
c) Reaktion des gemischten Anhydrids (IV) mit einem Phenol der allgemeinen Formel (V):

(V)

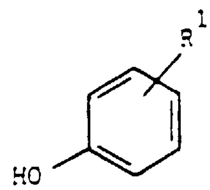

in der $R^1$ aus H und $C_1$-$C_5$ Alkyl, $C_2$-$C_5$ Acyl und einer Nitrogruppe ausgewählt wird, bei einer Temperatur unter 60°C in Anwesenheit eines aprotischen Lösungsmittels und eines tertiären Amins, um den entsprechenden Phenolester der Formel (VI):

(VI)

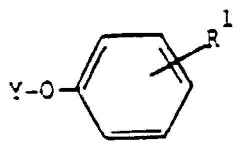

zu erhalten,
d) Trennung des Produktes durch Wasserzugabe, Phenolester-Extraktion in einem Lösungsmittel, Verdunstung und Kristallisierung, wahlweise von einer Wiederkristallisierung gefolgt;
e) Behandlung des Phenolesters mit einer wässerigen Lösung von Glyzin als solches oder in der Form eines alkalischen Metallsalzes oder in der Form eines tertiären Amins von Alkyl- oder heteroaromatischem Typ, bei einer Temperatur zwischen 0°C und 100°C, gegebenfalls in Anwesenheit eines protischen Lösungsmittels;
f) Trennung des Produktes, Ausfällung durch Ansäuern der unter e) erzeugten Reaktionsmischung zu einem pH zwischen 1 und 4, nach der wahlfreien Verdunstung des Lösungsmittels und dem zugehörigen Filtrieren;
g) das unter (f) erhaltene Produkt wird in einem protischen oder aprotischen polaren Lösungsmittel kristallisiert.

**2.** Verfahren nach Anspruch 1, in dem das in den Phasen (a) und (b) benutzte aprotische Lösungsmittel ein aprotisches polares Lösungsmittel ist, das aus Azeton, Äthylazetat, Dioxan, Tetrahydrofuran ausgewählt wird, oder ein aprotisches dipolares Lösungsmittel ist, das aus Dimethylformamid ausgewählt wird.

**3.** Verfahren nach Anspruch 1, in dem die in den Phasen (a) und (b) erhaltenen Zwischenprodukte nicht isoliert werden.

**4.** Verfahren nach Anspruch 1, in dem die tertiären Amine, die in den Phasen (a) und (c) benutzt wurden, um das Gallensäure-Salz, beziehungsweise das Phenolsalz zu erhalten, aus Triethylamin, Tributylamin und Pyridin ausgewählt werden.

**5.** Verfahren nach Anspruch 1, in dem das $C_1$-$C_5$ Alkyl-Chloroformiat (III), das in der Phase (b) benutzt wurde, Methyl- oder Äthyl-Chloroformiat ist.

**6.** Verfahren nach Anspruch 1, in dem das in der Phase (c) benutzte aprotische Lösungsmittel ein aprotisches polares

Lösungsmittel ist, das aus Azeton, Äthylazetat, Dioxan, Tetrahydrofuran ausgewählt wird, oder ein aprotisches dipolares Lösungsmittel ist, wie N,N-Dimethylformamid.

**7.** Verfahren nach Anspruch 1, in dem das Lösungsmittel zur Kristallisierung des Phenolesters in der Phase (d) ein protisches oder aprotisces polares Lösungsmittel ist, vorzugsweise Acetonitril, entweder allein oder mit $C_1$-$C_4$ Alkoholen gemischt.

**8.** Verfahren nach Anspruch 2, in dem die Basen, die benutzt wurden, um das in der Phase (e) benutzte Glyzinsalz zu erhalten, aus Natriumhydroxyd, Ätzkali, Lithiumhydroxyd, Kalium- oder Natriumkarbonat oder -bikarbonat ausgewählt werden, und die tertiären Amine aus Triethylamin, Tributylamin und Pyridin ausgewählt werden.

**9.** Verfahren nach Anspruch 1, in dem die in der Phase (f) für die Ausfällung der konjugierten rohen Säure (I) benutzte Säure eine Mineralsäure ist, die aus Chlorwasserstoffsäure, Schwefelsäure und Phosphorsäure ausgewählt wird, oder eine organische Säure ist, wie Essigsäure.

**10.** Verfahren nach Anspruch 1, in dem das in der Phase (g) für die Kristallisierung von konjugierter Gallensäure (I) benutzte Lösungsmittel, ein aus einem Alkohol bestehendes protisches polares Lösungsmittel, oder ein aus Azeton oder $C_1$-$C_4$ Alkoholazetaten bestehendes aprotisches polares Lösungsmittel ist.

## Revendications

**1.** Procédé de préparation des acides biliaires conjugés à glycine de formule générale (I):

(I) $Y\text{-}NH\text{-}CH_2\text{-}COOH$

où Y est l'acyle radical d'un acide biliaire choisi dans le groupe formé par les acides ursodéoxycholique, chénodéoxycholique, lithocholique, 3α-7β-12α-tri-hydroxycholanique, 3α-7β-dihydroxy-12-kétocholanique, déoxycholique, déhydrocholique, hyodéoxycholique, hyocholique, comprenant:

a) éventuellement salifier un acide biliaire de formule (II):

(II) Y-OH

où Y est comme ci-dessus, avec une amine tertiaire de type alkyle ou hétéroaromatique dans un solvant aprotique à une température inférieure à 20°C;

b) traiter le mélange de réaction comprenant le susdit sel de l'acide biliaire, ou le sel isolé précédemment, avec un chloroformiate de formule générale (III):

(III) Cl-COOR

où R est choisi dans le groupe formé par alkyle $C_1$-$C_5$ , phényl, benzyle, à une température inférieure à 20°C en présence d'un solvant aprotique pour donner l'anhydride mélangé correspondant de formule (IV):

(IV) Y-O-COOR

où Y et R sont comme ci-dessus;

c) réagir l'anhydride mélangé (IV) avec un phénol de formule générale (V):

(V) 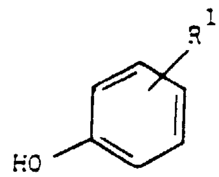

où $R^1$ est choisi dans le groupe formé par H et alkyle $C_1$-$C_5$ , acyle $C_2$-$C_5$, et groupe nitro, à une température inférieure à 60°C en présence d'un solvant aprotique et d'une amine tertiaire pour donner le phénol ester correspondant de formule (VI):

(VI) 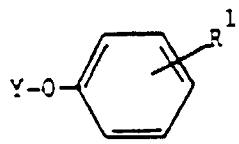

d) séparer le produit par adjonction d'eau, extraction du phénol ester dans un solvant, évaporation et cristallisation, éventuellement suivies par recristallisation;

e) traiter le phénol ester avec une solution aqueuse de glycine en tal que telle ou sous forme d'un sel métallique alcalin ou d'une amine tertiaire de type alkyle ou hétéroaromatique à une température de 0°C-100°C, éventuellement en présence d'un solvant protique;

f) séparer le produit, précipiter par acidification du mélange de réaction produit dans la phase (e) jusqu'à un pH entre 1 et 4, après un'évaporation optionelle du solvant et la relative filtration;

g) le produit recueilli dans la phase (f) est cristallisé dans un solvant polaire protique ou aprotique.

**2.** Procédé selon la revendication 1, dans lequel le solvant aprotique utilisé dans les phases (a) et (b) est un solvant polaire aprotique choisi dans le groupe formé par acétone, éthyl acétate, dioxane, tétrahydrofuran ou un solvant dipolaire aprotique choisi dans la diméthylformamide.

**3.** Procédé selon la revendication 1, dans lequel les intermédiaires obtenus dans (a) et (b) ne sont pas isolés.

**4.** Procédé selon la revendication 1, dans lequel les amines tertiaires utilisées dans (a) et (c) pour donner le sel de l'acide biliaire et le sel phénol, respectivement, sont choisies dans le groupe formé par triéthylamine, tributylamine et pyridine.

**5.** Procédé selon la revendication 1, dans lequel l'alkyl chloroformiate $C_1$-$C_5$ (III) utilisé dans (b) est méthyl- ou éthyl chloroformiate.

**6.** Procédé selon la revendication 1, dans lequel le solvant aprotique utilisé dans (c) est un solvant polaire aprotique choisi dans le groupe formé par acétone, éthyl acétate, dioxane, tétrahydrofuran, ou un solvant dipolaire aprotique, comme N,N-diméthylformamide.

**7.** Procédé selon la revendication 1, dans lequel le solvant de cristallisation du phénol ester de la phase (d) est un solvant polaire protique ou aprotique, de préférence acétonitrile, seul ou mélangé avec alcools $C_1$-$C_4$ .

**8.** Procédé selon la revendication 2, dans lequel les bases utilisées pour obtenir le sel de glycine utilisé dans la phase (e) sont choisies dans le groupe formé par: hydroxyde de sodium, hydroxyde de potassium, hydroxyde de lithium, carbonate ou bicarbonate de potassium ou de sodium, et les amines tertiaires sont choisies dans le groupe formé par triéthylamine, tributylamine et pyridine.

**9.** Procédé selon la revendication 1, dans lequel l'acide utilisé dans la phase (f) pour la précipitation de l'acide brut conjugué (I) est un acide minéral choisi dans le groupe formé par les acides chlorhydrique, sulfurique et phosphorique, ou un acide organique comme acide acétique.

**10.** Procédé selon la revendication 1, dans lequel le solvant utilisé pour la cristallisation de l'acide biliaire conjugué (I)

dans la phase (g) est un solvant polaire protique formé par un alcool ou un solvant polaire aprotique formé par acétone ou alcool acétates $C_1$-$C_4$ .